# EUROPEAN PATENT SPECIFICATION

(11) **EP 2 186 501 B1**
(45) Date of publication and mention of the grant of the patent: **01.08.2012**
(21) Application number: 08425731.0
(22) Date of filing: 14.11.2008
(51) Int. Cl.: A61H 23/02, A61N 7/00

(54) **Ultrasonic transducer including a diffuser**
Ultraschallwandler mit Diffusor
Transducteur ultrasonique avec diffuseur

(43) Date of publication of application: 19.05.2010
(73) Proprietor: Lain Electronic S.r.L., 20011 Corbetta MI (IT)
(72) Inventor: Marzorati, Armando, 20010 Santo Stefano Ticino (Milano) (IT)
(74) Representative: Lunati, Vittoriano

(56) References cited:
- EP-A- 1 844 750
- DE-A1- 10 153 126
- DE-U1- 20 313 649
- US-A1- 2006 090 956

## Description

The present invention relates to a transducer device including an improved diffuser of the type specified in the preamble of claim 1.

There are currently know transducer devices intended in particular for the treatment of cellulite and of the adipose tissue in the human body.

These devices are suitable to produce the known phenomenon of cavitation inside the human body, in particular in the adipose areas. Cavitation allows destruction of the adipose cells which are subsequently expelled by the body's normal metabolism in the form of liquid waste.

In particular, the transducer device comprises piezoelectric elements which are stimulated by alternating electric current.

The piezoelectric elements then transform the alternating electric current into alternating mechanical vibrations.

The mechanical vibrations are then transmitted to a diffuser, provided with a surface which is placed in contact with the skin in the adipose areas of the human body.

The diffuser then transmits the vibrations to the adipose tissues, causing said cavitation phenomenon.

A device of this kind is described, for example, in the patent application EP-A-1844750, by the same applicant, DE-A-10153126, DE-U-20313649 and US-A-2006/090956.

The document EP-A-1 844 750 discloses a transducer device according to the preamble of claim 1.

The prior art mentioned above has some limitations.

In fact, the vibrations are transmitted in particular to the surface of the human body. Therefore, it is difficult to destroy adipose tissue located below a certain depth, in particular depths greater than 2.5 cm.

In similar devices, attempts have been made to solve this drawback by means of transducer devices including a parabolic diffuser, which concentrates the vibrations in depth and not on the surface.

However, these diffusers present the drawback of having an excessively high temperature at the edges, where the vibrations are concentrated.

Therefore, they can burn the person undergoing treatment and, moreover, do not completely solve the aforesaid problems.

In this situation the technical aim underlying the present invention is to devise a transducer device including an improved diffuser capable of substantially overcoming the aforesaid drawbacks.

Within said technical aim, an important object of the invention is a transducer device including an improved diffuser suitable to allow deep penetration of the mechanical vibrations causing cavitation.

The technical aim and the object specified are achieved by a transducer device including an improved diffuser as claimed in the appended claim 1.

Preferred embodiments are defined in the dependent claims.

The features and advantages of the invention are better explained below by the detailed description of a preferred embodiment of the invention, with reference to the attached drawings, wherein:
**Fig. 1** shows a sagittal section of the transducer device according to the invention;
**Fig. 2** shows a side view of the transducer device according to the invention;
**Fig. 3** defines a three-dimensional view of the transducer device according to the invention; and
**Fig. 4** presents an operating diagram of the device according to the invention.

With reference to the aforesaid Figures, the transducer device according to the invention is indicated as a whole with the number **1.**

This device is suitable to generate cavitation in adipose tissue and the like if applied to the external surface of the human body, preferably by means of vibrations having frequencies of between 25 kHz and 50 kHz, and more preferably between 36 kHz and 42 kHz. Moreover, these frequencies vary continuously, as described in the application EP-A-1844750, in particular from paragraph [0041] to paragraph [0047].

The transducer device 1 is then connected to a generator apparatus, suitable to generate, or more simply to emit, alternating electric current and one or more transducers 1, electrically connected by means of specific electrical connections **2.**

A generator apparatus of this kind is described, for example, in the publication of aforesaid application EP-A-1844750, from paragraph [0048] to paragraph [0059] and illustrated in the relative figures.

This apparatus comprises control means suitable to adjust the electrical frequency and to activate, deactivate and in general control the transducer device **1.**

The transducer device 1 then transforms the alternating electric signal coming from the electrical connections 2 into mechanical vibrations with the same frequencies.

It presents resonance frequencies and, if stimulated at the same frequencies, causes particularly high amplitude mechanical vibrations, which allow the establishment of the cavitation phenomenon.

In particular, the transducer device 1 is realized in such as way as to obtain resonance frequencies corresponding to the 25 kHz to 50 kHz frequency interval, more particularly corresponding to the 36 kHz - 42 kHz frequency interval. In fact, whenever cavitation is produced within these vibration intervals, the positive effects of reducing adipose tissues and cellulite linked to cavitation are maximised, as described in the application EP-A-1844750.

The transducer device 1 also comprises a plurality of piezoelectric units **3.**

These piezoelectric units 3 are made from piezoelectric ceramic material, preferably realized by a lead-titanium intermetallic ceramic commonly known as "lead-titanate".

They are preferably disk-shaped, and are furthermore preferably arranged in stacks, and electrically connected to the electrical connectors 2. They preferably have external diameters of between 34 mm and 23 mm, and a height of between **4** mm and 9 mm.

The electrical connections 2 comprise conductor elements **4** and electrical cables **5,** reciprocally connected.

In particular, the former are suitable to supply the piezoelectric units 3 and the latter connect the conductor elements 4 to the generator apparatus.

The conductor elements 4 are appropriately disk-shaped and made from an alloy of copper or other conducting metals, and interposed between the stacked piezoelectric units 3. Moreover, they preferably have a diameter identical or similar to the diameter of the piezoelectric units 3 and a height of less than 4 mm.

The transducer device 1 preferably also comprises a block body **6,** also disk-shaped, and a diffuser 7, both made from metallic materials, preferably steel.

The block body 6 and the diffuser 7 are preferably stacked at the two ends of the piezoelectric units 3 and of the conductors **4,** and enclose these by means of specific fixing means **8,** appropriately realized by an Allen screw engaging with the diffuser 7.

With the aim of allowing fixing of the block body 6, of the piezoelectric units 3, of the conductors 4 and of the diffuser 7, these components have a central through-hole, through which the fixing means 8 pass.

In particular, the fixing means 8 engage with the block body 6, appropriately by means of the head of the screw, having dimensions greater than the dimensions of the through-hole formed in the block body 6, and with the diffuser 7 presenting a blind threaded hole. Therefore, the fixing means 8 sandwich the various components of which the transducer 1 is composed.

The block body 6 and the fixing means 8, have the purpose of allowing correct channelling of the mechanical vibrations towards the diffuser 7, which has the purpose of diffusing the mechanical vibrations produced by the piezoelectric units 3. In particular, the block body 6 has a cylindrical shape, a diameter similar or identical to the diameter of the piezoelectric units 3 and a height of between 4 mm and 10 mm.

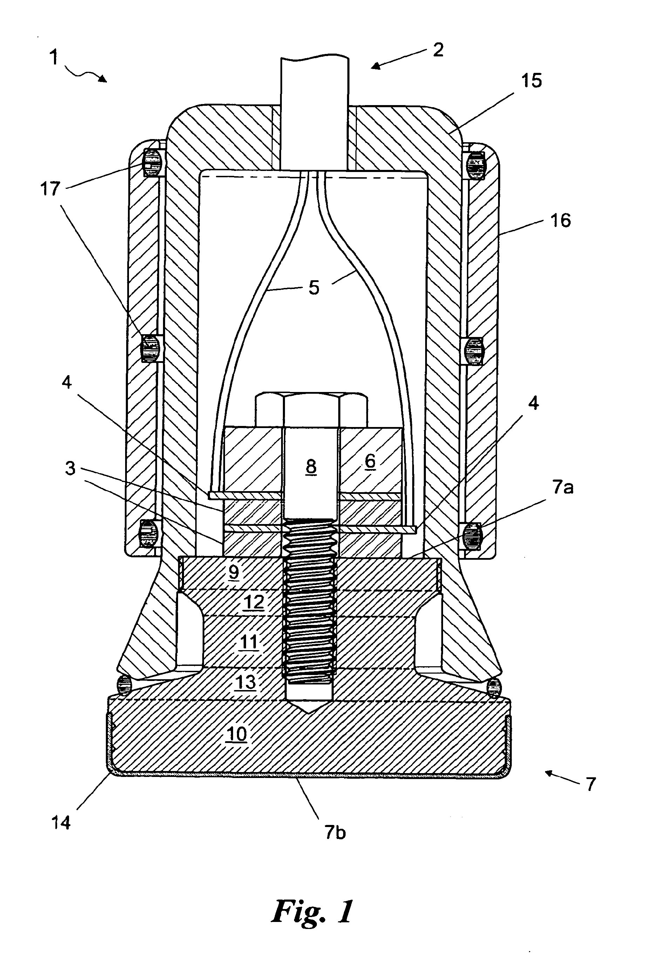

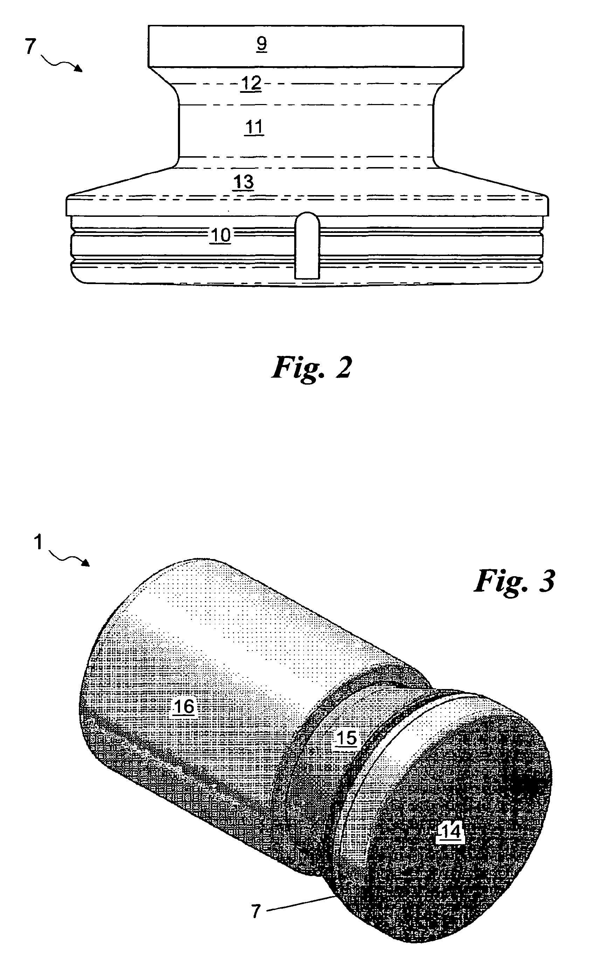

The diffuser 7 has a substantially axial symmetric structure with respect to a principal axis of extension **1a** coincident with the axis of the piezoelectric units 3 and comprising an upper face **7a,** in contact with said piezoelectric units 3, and a circular base face **7b,** opposite the upper face 7a. This is shown in Figs. 1 and 2.

The diffuser 7 comprises a first portion **9,** positioned in proximity to, and preferably including, the upper face 7a, substantially cylindrical and having a first diameter.

Moreover, the first diameter is smaller than the diameter of the base face 7b.

The diffuser 7 then comprises a terminal portion **10,** including the base face 7b, and also cylindrical and having a diameter equal to the diameter of the base face 7b.

Moreover, a second portion **11,** also substantially cylindrical and having a second diameter smaller than the first diameter, is present between the two portions 9 and 10.

A first and a second coupling portion **12** and **13** are also present, having a substantially truncated cone shaped structure and suitable to respectively couple the first and second portion 9 and 11 and the second portion 11 with the terminal portion 10.

In detail, the height of the diffuser 7 is between 25 mm and 41 mm.

The diameter of the base face 7b is preferably between 45 mm and 65 mm and more preferably between 56 mm and 60 mm, while the height of the terminal portion 10 is between 7 mm and 16 mm and more preferably between 10 mm and 13 mm.

The first diameter is preferably between 33 mm and 45 mm and more preferably between 36 mm and 42 mm, while the height of the first portion 9 is between 2 mm and 8 mm and more preferably between 4 mm and 6 mm.

The second diameter is preferably between 26 mm and 35 mm and more preferably between 29 mm and 33 mm, while the height of the second portion 11 is between 3 mm and 9 mm and more preferably between 6 mm and 8 mm.

In Fig. 1 the diffuser 4 is represented in scale.

Finally, the first portion 9 comprises a thread for coupling with a housing as described below, while the terminal portion 10 comprises a removable cap **14** made from metal and preferably from AISI 316-L stainless steel, and suitable to realize the element in contact with the skin of the patient.

This cap **14** can be constrained to the terminal portion 10 through known coupling means and allows the possible choice of an optimal material for contact with the skin and possible replacement thereof.

The device 1 also comprises a cylindrical receptacle **15,** manually grippable.

It houses the piezoelectric units 3, part of the electrical connections 2, the block body 6 and is connected to the diffuser 7, in particular to the thread present on the first portion 9.

The receptacle is also appropriately connected also to the terminal portion 10, not in direct contact but by means of an O-ring, as shown in Fig. 1.

Finally, the device 1 comprises an external handpiece **16,** cylindrical and connected to the receptacle 15 by means of vibration damping devices **17,** such as O-rings and the like.

Operation of a transducer device 1, the structure of which is described above, is as follows.

The generator apparatus, connected to the transducer device 1, is activated and the operator who administers the treatment to the patient holds the transducer device 1, by means of the external handpiece 16.

The diffuser 7 is placed in contact with the epidermis 3 of the patient undergoing treatment, in the areas affected by adipose tissues or cellulite.

An aqueous contact gel, in which the ultrasonic frequencies propagate, is spread on the epidermis of the patient in advance.

The transducer 1 transmits the ultrasounds inside the adipose tissues or cellulite, causing cavitation therein, and hence their destruction.

In particular, according to studies by the applicant, the vibrations form in the first and second portion 9 and 11 and pick up speed in said portions, which are placed at the centre of the normal section of the diffuser. In fact, these portions act as cavitation accelerators.

Subsequently, some vibrations expand to the peripheral parts, present only in the terminal portion 10. However, in these peripheral areas the vibrations have a lower speed and intensity compared to the central area.

Therefore, the vibrations are transmitted to the epidermis with greater intensity in the central area compared to the peripheral area and thus tend to be concentrated in depth in a first area, as shown in Fig. 2.

In a second area of the adipose tissue, at a greater depth with respect to the first area, the vibrations expand according to conventional wave propagation mechanics.

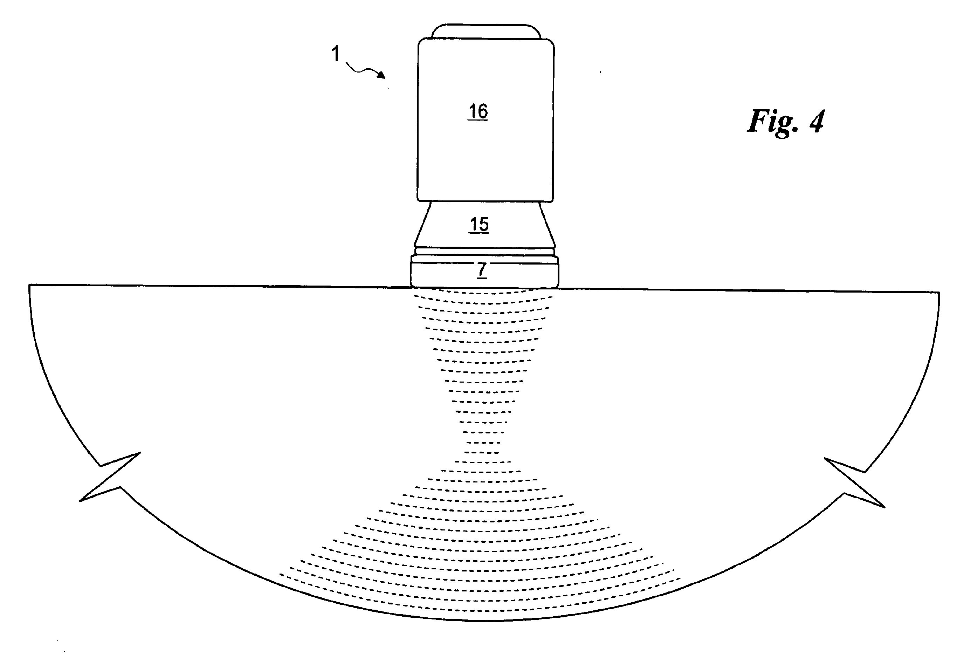

Different experiments of the applicant have in fact proved a propagation of the waves similar to that shown in Fig. 4.

The invention achieves important advantages.

In fact, the vibrations produced by the transducer device 1 penetrate in depth and cause cavitation in particular inside the adipose area.

Moreover, owing to the particular structure of the device 1 less energy per surface unit is necessary, in view of the high efficiency thereof.

Said advantages are determined in particular by the shape of the diffuser 7.

The invention is subject to variations falling within the scope of the claims.

## Claims

1. Transducer device (1) suitable to be connected to a generator apparatus of alternating electric current and comprising: at least one piezoelectric unit (3), suitable to transform said alternating electric current into mechanical vibrations having frequencies of between 25 kHz and 60 kHz, electrical connections (2) suitable to connect said at least one piezoelectric unit (3) to said generator apparatus, a diffuser (7) with a substantially axial symmetric structure and comprising an upper face (7a), in contact with said piezoelectric units (3), and a base face (7b) opposite said upper face (7a), said diffuser (7) comprises a substantially cylindrical terminal portion (10) including said base face (7b), a first portion (9) placed in proximity of said upper face (7a) and substantially cylindrical and provided with a first diameter smaller than the diameter of said base face (7b); **characterized in that** said diffuser (7) comprise a second portion (11), placed between said first portion (9) and terminal portion (10), substantially cylindrical and having a second diameter, smaller than said first diameter and a first and a second coupling portion (12, 13) having a substantially truncated cone shaped structure and suitable to respectively couple said first and second portion (9, 11) and said second portion (11) and terminal portion (10).

2. Transducer device (1) according to claim 1, wherein said first portion (9) comprises said upper face (7a).

3. Transducer device (1) according to one or more of the preceding claims, wherein said terminal portion (10) comprises a removable cap (14) suitable to realize an element in contact with the skin of the patient.

4. Transducer device (1) according to one or more of the preceding claims, wherein the height of said diffuser (7) is between 25 mm and 41 mm and the diameter of said base face (7b) is between 45 mm and 65 mm.

5. Transducer device (1) according to one or more of the preceding claims, comprising a cylindrical receptacle (15) housing said piezoelectric units (3), part of said electrical connections (2), and constrained to said diffuser (7).

6. Transducer device (1) according to claim 5, wherein said cylindrical receptacle (15) is connected to said diffuser (7) by means of a thread present on said first portion (9).

7. Transducer device (1) according to claim 5 or 6, comprising an external handpiece (16), cylindrical and connected to said receptacle (15) by means of vibration damping devices (17), such as O-rings and the like.

## Patentansprüche

1. Umwandlervorrichtung (1), welche zum Anschluss an einen Wechselstromgenerator geeignet ist und folgendes aufweist:
mindestens eine piezoelektrische Einheit (3) mit zwischen 25 kHz und 50 kHz liegenden Frequenzen, die zur Umwandlung des genannten Wechselstroms in mechanische Schwingungen geeignet ist, Stromanschlüsse (2), die zum Anschluss der genannten mindestens einen piezoelektrischen Einheit (3) an den genannten Generator geeignet sind, einen Verteiler (7) mit im Wesentlichen achsensymmetrischer Struktur, der eine an die genannten piezoelektrischen Einheiten (3) anliegende Oberfläche (7a) und eine der genannten Oberfläche (7a) entgegengesetzte Grundfläche (7b) aufweist, wobei der genannte Verteiler (7) einen im Wesentlichen zylinderförmigen, die genannte Grundfläche (7b) einschließenden Endabschnitt (10) umfasst, sowie einen in unmittelbarer Nähe der genannten Oberfläche (7a) liegenden, im Wesentlichen zylinderförmigen ersten Abschnitt (9), dessen erster Durchmesser geringer als der Durchmesser der genannten Grundfläche (7b) ist; **dadurch gekennzeichnet, dass** der genannte Verteiler (7) einen zwischen den genannten ersten Abschnitt (9) und Endabschnitt (10) liegenden, im Wesentlichen zylinderförmigen zweiten Abschnitt (11) umfasst, dessen zweiter Durchmesser geringer als der genannte erste Durchmesser ist, sowie einen ersten und zweiten Verbindungsabschnitt (12, 13) mit im Wesentlichen kegelstumpfiger Struktur, die jeweils zur Verbindung des genannten ersten und zweiten Abschnitts (9, 11) und des genannten zweiten Abschnitts (11) mit dem Endabschnitt (10) geeignet sind.

2. Umwandlervorrichtung (1) nach Anspruch 1, wobei der genannte erste Abschnitt (9) die genannte Oberfläche (7a) einschließt.

3. Umwandlervorrichtung (1) nach einem oder mehrerer der vorhergehenden Ansprüche, wobei der genannte Endabschnitt (10) eine trennbare Kappe (14) aufweist, die als Kontaktelement zur Haut des Patienten dient.

4. Umwandlervorrichtung (1) nach einem oder mehrerer der vorhergehenden Ansprüche, wobei die Höhe des genannten Verteilers (7) zwischen 25 mm und 41 mm und der Durchmesser der genannten Grundfläche (7b) zwischen 45 mm und 65 mm liegt.

5. Umwandlervorrichtung (1) nach einem oder mehrerer der vorhergehenden Ansprüche, welche einen zylinderförmigen Behälter (15), in dem die genannten piezoelektrischen Einheiten (3) untergebracht sind, sowie einen Teil der genannten Stromanschlüsse (2) aufweist, und der mit dem genannten Verteiler (7) verbunden ist.

6. Umwandlervorrichtung (1) nach Anspruch 5, wobei der genannte zylindeförmige Behälter (15) mit dem genannten Verteiler (7) durch ein im genannten ersten Abschnitt (9) vorhandenen Gewinde verbunden ist.

7. Umwandlervorrichtung (1) nach Anspruch 5 oder 6, welche ein zylinderförmiges Außenhandstück (16) aufweist, das mit dem genannte Behälter (15) mittels Dämpfern (17) wie z.B. O-ring o.ä. verbunden ist.

## Revendications

1. Dispositif transducteur (1) apte à être relié à un appareil générateur de courant électrique alternatif et comprenant: au moins une unité piézoélectrique (3) ayant des fréquences comprises entre 25 kHz et 50 kHz, destinée à convertir ledit courant électrique alternatif en vibrations mécaniques, des connexions électriques (2) aptes à connecter ladite au moins une unité piézoélectrique (3) audit appareil générateur, un diffuseur (7) de structure sensiblement symétrique en sens axial et comprenant une face supérieure (7a), au contact desdites unités piézoélectriques (3) et une face de base (7b) opposée à ladite face supérieure (7a), ledit diffuseur (7) comprenant une portion terminale (10) sensiblement cylindrique incluant ladite face de base (7b), une première portion (9) mise à proximité de ladite face supérieure (7a) et sensiblement cylindrique et ayant un premier diamètre inférieur au diamètre de ladite face de base (7b); **caractérisé en ce que** ledit diffuseur (7) comprend une deuxième portion (11), placée entre lesdites première portion (9) et portion terminale (10), sensiblement cylindrique et ayant un deuxième diamètre, inférieur audit premier diamètre et une première et une deuxième portions de raccordement (12, 13) de structure essentiellement tronconique et aptes à raccorder respectivement lesdites première et deuxième portions (9, 11) et lesdites deuxième portion (11) et portion terminale (10).

2. Dispositif transducteur (1) selon la revendication 1, dans lequel ladite première portion (9) comprend ladite face supérieure (7a).

3. Dispositif transducteur (1) selon une ou plusieurs des revendications précédentes, dans lequel ladite portion terminale (10) comprend un capuchon (14) séparable, apte à former un élément au contact de la peau du patient.

4. Dispositif transducteur (1) selon une ou plusieurs des revendications précédentes, dans lequel la hauteur dudit diffuseur (7) est comprise entre 25 mm et 41 mm et le diamètre de ladite face de base (7b) est compris entre 45 mm et 65 mm.

5. Dispositif transducteur (1) selon une ou plusieurs des revendications précédentes, comprenant un conteneur cylindrique (15) logeant lesdites unités piézoélectriques (3), une partie desdites connexions électriques (2) et fixé audit diffuseur (7).

6. Dispositif transducteur (1) selon la revendication 5, dans lequel ledit conteneur cylindrique (15) est relié audit diffuseur (7) par l'intermédiaire d'un filet présent sur ladite première portion (9).

7. Dispositif transducteur (1) selon la revendication 5 ou 6, comprenant une poignée extérieure (16), cylindrique et reliée audit conteneur (15) à l'aide d'amortisseurs (17) tels que des garnitures de type "O-ring" et similaires.
